(19) Europäisches Patentamt
European Patent Office
Office européen des brevets

(11) Veröffentlichungsnummer: **0 075 149**
**A2**

(12) **EUROPÄISCHE PATENTANMELDUNG**

(21) Anmeldenummer: **82108040.5**

(22) Anmeldetag: **01.09.82**

(51) Int. Cl.³: **C 07 D 271/06**
// C07D317/30, C07D413/06

(30) Priorität: **05.09.81 DE 3135237**

(43) Veröffentlichungstag der Anmeldung: **30.03.83**
**Patentblatt 83/13**

(84) Benannte Vertragsstaaten: **CH DE FR GB IT LI NL**

(71) Anmelder: **HOECHST AKTIENGESELLSCHAFT,**
**Postfach 80 03 20, D-6230 Frankfurt/Main 80 (DE)**

(72) Erfinder: **Kübel, Börries, Dr., Kuckucksweg 14,**
**D-6233 Kelkheim (Taunus) (DE)**

(54) **Substituierte 1.2.4-Oxadiazole und Verfahren zu ihrer Herstellung.**

(57) Gegenstand der Erfindung sind Verbindungen der Formel I

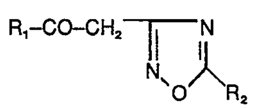

(I)

worin

$R_1$ = Wasserstoff oder $CH_3$ und $R_2$ Wasserstoff, gegebenenfalls substituiertes Alkyl, Cycloalkyl oder gegebenenfalls substituiertes Phenyl bedeuten, Verfahren zu deren Herstellung und ihre Verwendung zur Herstellung von Pflanzenschutzmitteln.

EP 0 075 149 A2

0075149

Substituierte 1.2.4-Oxadiazole und Verfahren zu ihrer Herstellung

Gegenstand der Erfindung sind die neuen substituierten 1.2.4-Oxadiazole der Formel

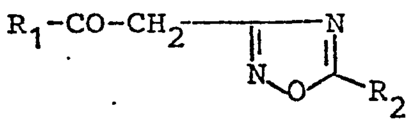
(I)

worin

$R_1$ Wasserstoff oder $CH_3$ und

$R_2$ Wasserstoff, $C_1$-$C_6$-Alkyl, das gegebenenfalls auch durch Halogen, $C_1$-$C_3$-Alkoxy, $C_1$-$C_3$-Alkylthio, Di-($C_1$-$C_3$-alkyl)-amino oder Phenyl substituiert sein kann; $C_3$-$C_6$-Cycloalkyl oder gegebenenfalls durch $C_1$-$C_3$-Alkyl, $C_1$-$C_3$-Alkoxy, Halogen oder Nitro substituiertes Phenyl bedeuten.

Gegenstand der Erfindung ist weiterhin ein Verfahren zur Herstellung von Verbindungen der Formel I, dadurch gekennzeichnet, daß man Verbindungen der Formel

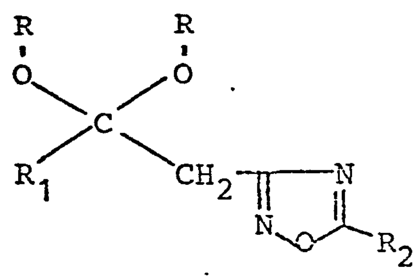
(II)

worin R Methyl oder Ethyl oder beide zusammen eine $C_2$-$C_3$-Alkylengruppe bedeuten, unter sauren Bedingungen hydrolysiert. Als Hydrolysemedium kommen verdünnte Mineralsäuren, saure Ionenaustauscher, wäßrige oder wasserfreie Ameisensäure, Essigsäure, Trifluoressigsäure oder Oxalsäure gegebenenfalls in Mischung mit Mineralsäuren und/oder mit Lösungsmitteln wie niederen Alkoholen, Ketonen, Glykolethern, THF, Dioxan, Acetonitril, Toluol, Hexan, Methylenchlorid und

BAD ORIGINAL

dgl. in Betracht. Die Reaktionstemperatur kann von 0°C bis zur Rückflußtemperatur des Gemisches variiert werden. Die Aufarbeitung erfolgt in üblicher Weise, z.B. durch Eindampfen und Destillieren des Produktes.

Verbindungen der Formel II erhält man aus 3.3-Dialkoxypropion- oder -butyramidoximen der Formel

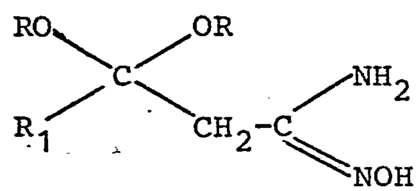

(III)

durch Ringschluß mit Carbonestern der Formel

$R_2-CO_2Alkyl$ (IV)

in alkoholischer Alkoholatlösung (vgl. J. Chem. Res. S 1979, 64 - 65 und Arzneimittelforschung 16 (1966), 615). Die Verbindungen der Formel III sind bekannt (DT-OS 28 13 340) bzw. lassen sich nach bekannten Verfahren herstellen.

Die erfindungsgemäßen Verbindungen der Formel I sind Zwischenprodukte für insektizide Phosphorester gemäß Patentanmeldung

Folgende Beispiele sollen die Erfindung verdeutlichen.

Beispiel 1
3-Acetonyl-5-methyl-1.2.4-oxadiazol

a) 3.3-Ethylendioxybutyramidoxim:
82 g (1 mol) 3-Aminocrotonsäurenitril werden unter Kühlung mit 100 ml Wasser und 100 ml konz. Salzsäure versetzt, 1 1/2 h bei 80°C gerührt, rasch abgekühlt und mit 100 ml Toluol versetzt. Man filtriert, trennt vom Filtrat die organische Phase ab, extrahiert die Wasserphase noch zweimal mit je 100 ml Toluol und trocknet die vereinten

organischen Phasen. Zu dieser Lösung werden 62 g (1 mol) Ethylenglykol und 0.5 g p-Toluolsulfonsäure zugegeben, dann wird die Mischung 4 h am Wasserabscheider gekocht. Anschließend dampft man im Retationsverdampfer ein und destilliert den Rückstand im Vakuum. Man erhält 107 g (85 %) 3.3-Ethylendioxybutyronitril, Siedepunkt 86 - 89°C/10 mbar.

Dieses Produkt fügt man zu einer unter Eiskühlung hergestellten Lösung aus 80 g (2 mol) Natriumhydroxid und 114 g (1.65 mol) Hydroxylammoniumchlorid in 400 ml Wasser und 400 ml Methanol. Man läßt über Nacht stehen, rührt noch 2 h unter Rückfluß und extrahiert dann dreimal mit Ethylacetat, trocknet die Extrakte und dampft im Vakuum ein. Ausbeute 96 g (71 %) Öl, das langsam fest wird. Schmelzpunkt 67°C.

b) 3-(2.2-Ethylendioxypropyl)-5-methyl-1.2.4-oxadiazol:

Man löst 5.75 g Natrium in 75 ml Ethanol und fügt eine Lösung von 16 g (0.1 mol) des unter a) erhaltenen Amidoxims und 26.4 mol (0.3 mol) Ethylacetat in 250 ml Ethanol zu. Dann rührt man 6 h unter Rückfluß und dampft anschließend im Rotationsverdampfer ein. Der Rückstand wird in Wasser gelöst und dreimal mit Methylenchlorid extrahiert; die Extrakte werden getrocknet und im Vakuum eingedampft. Ausbeute 15.3 g (83 %) orangefarbenes Öl.
$^1$H-NMR ($CDCl_3$): $\delta$= 1.45 (s; 3 H), 2.55 (s; 3 H), 3.05 (s; 2 H), 4.0 (s; 4 H).

BAD ORIGINAL

c) 3-Acetonyl-5-methyl-1.2.4-oxadiazol:

Das unter b) erhaltene Produkt (0.083 mol) wird mit 40 ml 2 N Schwefelsäure und 25 ml Ethanol versetzt und 14 h bei 80°C gerührt. Dann gießt man auf 150 ml Wasser, extrahiert dreimal mit Methylenchlorid, trocknet, dampft im Vakuum ein und destilliert den Rückstand im Vakuum. 5.3 g (46 %) farblose Flüssigkeit mit Siedepunkt 63 - 67°C/0.4 mbar.

Beispiel 2:

3-Acetonyl-5-ethyl-1.2.4-oxadiazol

a) 5-Ethyl-3-(2.2-ethylendioxypropyl)-1.2.4-oxadiazol:

Es wurde aus 3.3-Ethylendioxybutyramidoxim und Propionsäureethylester analog zu Beispiel 1 b) hergestellt, oranges Öl, 67 % Ausbeute.

b) 19.8 g (0.1 mol) des nach 2 a) erhaltenen Produktes werden mit 60 g Ionenaustauscher Merck I (stark sauer) in 200 ml Hexan 3 h unter Rückfluß gerührt. Nach dem Abkühlen saugt man von Ionenaustauscher ab, neutralisiert diesen mit gesättigter Natriumhydrogencarbonatlösung und wäscht ihn mit Ethylacetat aus. Das Hexanfiltrat und die Ethylacetatlösung werden vereinigt, im Vakuum eingedampft und destilliert. 5.84 g (38 %) farblose Flüssigkeit mit Siedepunkt 82 - 83°C/0.4 mbar.

Beispiel 3

3-Acetonyl-5-isopropyl-1.2.4-oxadiazol:

a) 3-(2.2-Ethylendioxypropyl)-5-isopropyl-1.2.4-oxadiazol:

Es wurde aus 3.3-Ethylendioxybutyramidioxim, Isobuttersäuremethylester und Natrium in Ethanol analog zu Beispiel 1 b) hergestellt, wobei das Molverhältnis Amidoxim: Ester: Natrium jedoch 1 : 2 : 2 betrug. Ausbeute 94 %, hellgelbes Öl.

b) 35 g (0.165 mol) des unter a) erhaltenen Produktes werden in 130 ml 98 %iger Ameisensäure mit 5 Tropfen 48 %iger Bromwasserstoffsäure versetzt und 1 h unter Rückfluß gerührt. Dann entfernt man die Ameisensäure am Rotationsverdampfer und destilliert den Rückstand im Vakuum. 16,5 g (60 %) farblose Flüssigkeit mit Siedepunkt 74°C/0.4 mbar.

BAD ORIGINAL

Analog zu den obigen Beispielen können folgende Verbindungen I erhalten werden:

| Beispiel | $R_1$ | $R_2$ | Siedepunkt ( °C/mbar) |
|---|---|---|---|
| 4 | $CH_3$ | H | |
| 5 | $CH_3$ | $CH_3OCH_2$- | 100/0.25 |
| 6 | $CH_3$ | $CF_3$- | 40/0.4 |
| 7 | $CH_3$ | n-$C_3H_7$- | |
| 8 | $CH_3$ | n-$C_4H_9$- | |
| 9 | $CH_3$ | i-$C_4H_9$- | |
| 10 | $CH_3$ | n-$C_6H_{13}$- | |
| 11 | $CH_3$ | Cyclo-$C_3H_5$- | 100 - 110/1.3 |
| 12 | $CH_3$ | Cyclo-$C_6H_{11}$- | 112 - 115/0.65 |
| 13 | $CH_3$ | $CH_3OCH_2CH_2$- | |
| 14 | $CH_3$ | $C_2H_5SCH_2CH_2$- | |
| 15 | $CH_3$ | $(CH_3)_2NCH_2CH_2$- | |
| 16 | $CH_3$ | $C_6H_5$- | |
| 17 | $CH_3$ | 4-$CH_3OC_6H_4$- | |
| 18 | $CH_3$ | 2-Cl-$C_6H_4$ - | |
| 19 | $CH_3$ | 3-$NO_2$-$C_6H_4$- | |
| 20 | $CH_3$ | 4-i-$C_3H_7$-$C_6H_4$- | |
| 21 | $CH_3$ | $C_6H_5CH_2$ - | 135 - 145/0.65 |
| 22 | H | $CH_3$- | |
| 23 | H | n-$C_3H_7$- | |

0075149

Patentansprüche:

1. Verbindungen der Formel I

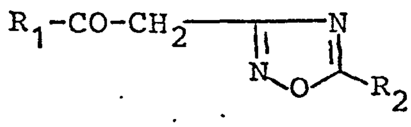

(I)

worin

$R_1$ Wasserstoff oder $CH_3$ und

$R_2$ Wasserstoff, $C_1$-$C_6$-Alkyl, das gegebenenfalls auch durch Halogen, $C_1$-$C_3$-Alkoxy, $C_1$-$C_3$-Alkylthio, Di-($C_1$-$C_3$-alkyl)amino oder Phenyl substituiert sein kann; $C_3$-$C_6$-Cycloalkyl oder gegebenenfalls durch $C_1$-$C_3$-Alkyl, $C_1$-$C_3$-Alkoxy, Halogen oder Nitro substituiertes Phenyl bedeuten.

2. Verfahren zur Herstellung von Verbindungen der Formel I, dadurch gekennzeichnet, daß man Verbindungen der Formel

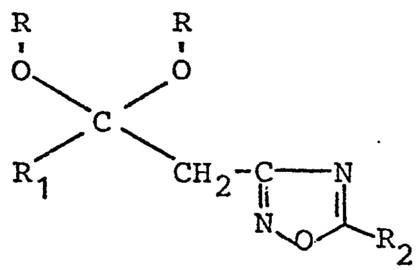

(II)

worin R Methyl oder Ethyl oder beide zusammen eine $C_2$-$C_3$-Alkylengruppe bedeuten, unter sauren Bedingungen hydrolysiert.

3. Verwendung von Verbindungen der Formel I als Ausgangsstoffe für die Herstellung von Pflanzenschutzmitteln.